# EUROPÄISCHE PATENTSCHRIFT

(11) **EP 0 866 679 B1**
(45) Veröffentlichungstag und Bekanntmachung des Hinweises auf die Patenterteilung: **08.09.1999**
(21) Anmeldenummer: 96945543.5
(22) Anmeldetag: 01.11.1996
(51) Int. Cl.: A61F 6/14

(54) **INTRAUTERINES PESSAR ZUR EMPFÄNGNISVERHÜTUNG**
INTRAUTERINE CONTRACTEPTIVE PESSARY
PESSAIRE INTRA-UTERIN CONTRACEPTIF

(30) Priorität: 01.11.1995 DE 19542386
(43) Veröffentlichungstag der Anmeldung: 30.09.1998
(73) Patentinhaber: Hamann, Bernd, 13187 Berlin (DE)
(72) Erfinder: Hamann, Bernd, 13187 Berlin (DE)
(74) Vertreter: Jander, Dieter, Dipl.-Ing.
(86) Internationale Anmeldenummer: DE9602124
(87) Internationale Veröffentlichungsnummer: WO9716142

(56) Entgegenhaltungen:
- WO-A-90/02478
- WO-A-90/09158
- FR-A- 2 116 174
- FR-A- 2 521 853
- US-A- 3 563 235
- US-A- 3 854 475
- US-A- 4 142 526
- US-A- 4 932 421
- US-A- 5 146 931

## Beschreibung

Die Erfindung bezieht sich auf ein intrauterines Pessar zur Empfängsnisverhütung, bestehend aus einem Stiel, zwei von diesem an seinem einen Ende nach zwei Seiten abragenden ersten Armen, zwei von dem Stiel an demselben Ende nach zwei Seiten abragenden zweiten Armen, die in den Winkeln zwischen den ersten Armen und dem Stiel angeordnet sind, und einer Drahtwicklung auf dem Stiel.

Bei einem bekannten Pessar dieser Art (FR-A-2521853) befinden sich auf den Armen keine Drahtwicklungen. Zwischen den Armen und dem Stiel bilden sich infolgedessen keine elektrolytischen Systeme aus. Die Empfängnisverhütung ist insofern eine entsprechend begrenzte.

Der Erfindung liegt die Aufgabe zugrunde, das eingangs erwähnte Pessar derart auszubilden, daß die Empfängnisverhütung besser als im bekannten Fall ist.

Diese Aufgabe wird erfindungsgemäß dadurch gelöst, daß die zweiten Arme Drahtwicklungen weitgehend über ihre gesamten Längen und die ersten Arme Drahtwicklungen aufweisen und daß die Metalle dieser Drahtwicklungen und der Drahtwicklung auf dem Stiel derart sind, daß zwischen zwei benachbarten Drahtwicklungen ein elektrolytisches System gebildet wird.

Auf diese Weise werden zwischen den ersten Armen und den zweiten Armen einerseits und den zweiten Armen und dem Stiel andererseits elektrolytische Systeme bzw. elektrische Felder gebildet, die die Empfängnisverhütung verbessern. Der Grund dafür ist darin zu sehen, daß ein elektrolytisches System bzw. ein elektrisches Feld die weißen Blutkörperchen aktiviert, die ihrerseits die Samenzellen schwächen oder zerstören. Dadurch, daß die Wicklungen auf den zweiten Armen weitgehend über die gesamte Länge der Arme reichen, wird unabhängig von der Länge und Lage der Drahtwicklungen auf den ersten Armen bzw. auf dem Stiel ein großflächiger Bereich in der Gebärmutter überdeckt.

Es sei erwähnt, daß auch schon ein Pessar bekannt ist (FR-A-2 116 174), bestehend aus einem Stiel, zwei von diesem an seinem Ende nach zwei Seiten abragenden ersten Armen, einer Drahtwicklung auf dem Stiel und Drahtwicklungen auf den ersten Armen, wobei die Metalle der Drahtwicklungen derart unterschiedlich sind, daß zwischen den Drahtwicklungen der Arme und des Stiels jeweils ein elektrolytisches System gebildet wird. Dieses Pessar besitzt jedoch nicht die zweiten Arme und Drahtwicklungen auf diesen mit der Maßgabe, daß zwischen zwei benachbarten Drahtwicklungen ein elektrolytisches System gebildet wird.

Ferner wird vorgeschlagen, daß die Drahtwicklungen auf den Armen weitgehend über die gesamten Längen der Arme reichen. Auf diese Weise wird erreicht, daß ein großer Bereich, vorzugsweise der gesamte Bereich des Eileitermündungsgebietes erfaßt wird, also der Bereich, wo sich die Eier ablagern.

Eine Weiterentwicklung der Erfindung besteht darin, daß die Drahtwicklung auf dem Stiel weitgehend über dessen gesamte Länge reicht. Auf diese Weise wird die durch das elektrische Feld erfaßte Fläche vergrößert.

Sodann wird vorgeschlagen, daß die Arme über ihre gesamte Länge zum Stiel hin leicht gebogen sind. Der Vorteil dieser Maßnahme besteht darin, daß auf diese Weise die Arme sich dem Eileitermündungsgebiet besonders gut anpassen.

Weiterhin wird erfindungsgemäß vorgeschlagen, daß die ersten Arme und der Stiel mit Kupfer und die zweiten Arme mit Zink umwickelt sind. Diese Metalle wirken sich besonders günstig empfängnisverhütend aus. Hinzu kommt, daß sich Kupfer und Zink außerdem auch in gesundheitlicher und sexueller Hinsicht günstig auswirken.

Natürlich ist es erfindungsgemãß auch möglich, daß die ersten Arme und der Stiel mit Zink und die zweiten Arme mit Kupfer umwickelt sind.

Sodann wird vorgeschlagen, daß die Drähte einen Silber-Kern aufweisen. Auf diese Weise wird erreicht, daß die Drähte einerseits haltbar und andererseits biegsam sind.

Anstelle von Zink kann erfindungsgemäß auch Gold vorgesehen werden. In diesem Falle könnte der Silber-Kern entfallen.

Schließlich wird vorgeschlagen, daß sich in den - vorzugsweise kugeligen - Enden der Arme und in dem - vorzugsweise kugeligen - Ende des Stiels, von dem die Arme abgehen, ein Hormon befindet, das eine empfängnisverhütende Wirkung hat und über eine semipermeable Membran nach außen dringen kann. Dadurch wird die empfängnisverhütende Wirkung weiter verbessert.

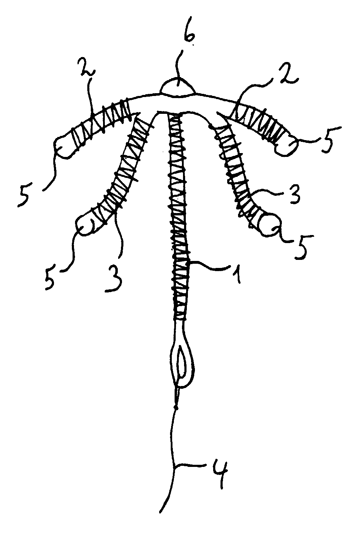

Weitere Einzelheiten der Erfindung ergeben sich aus der einzigen Figur der Zeichnung, in der ein Pessar gemäß der Erfindung veranschaulicht ist.

Das Pessar besteht aus einem Stiel 1, zwei ersten Armen 2 und zwei zweiten Armen 3. Der Stiel 1 und die Arme 2 und 3 sind jeweils von einer Metallwicklung umgeben. Am unteren Ende des Pessars befindet sich eine Schnur 4 zur Handhabung des Pessars. In den kugeligen Enden 5 der Arme 2,3 und in dem Kopf 6 am oberen Ende des Stiels 1 befindet sich ein Hormon, das über semipermeable Wände nach außen dringen kann.

Die Tatsache, daß die zweiten Arme 3 unten, innen von den Armen 2 abgehen, fuhrt dazu, daß die Bewegungen, die die Arme 2 durch die Gebärmutter erfahren, zu Bewegungen der Arme 3 fuhren, was sich günstig auf die Empfängnisverhütung auswirkt.

## Patentansprüche

1. Intrauterines Pessar zur Empfängnisverhütung, bestehend aus einem Stiel (1), zwei von diesem (1) an seinem einen Ende nach zwei Seiten abragenden ersten Armen (2), zwei von dem Stiel (1) an demselben Ende nach zwei Seiten abragenden zweiten Armen (3), die in den Winkeln zwischen den ersten Armen (2) und dem Stiel (1) angeordnet sind, und einer Drahtwicklung auf dem Stiel, **dadurch gekennzeichnet**, daß die zweiten Arme (3) Drahtwicklungen weitgehend über ihre gesamten Längen und die ersten Arme (2) Drahtwicklungen aufweisen und daß die Metalle dieser Drahtwicklungen und der Drahtwicklung auf dem Stiel (1) derart sind, daß zwischen zwei benachbarten Drahtwicklungen ein elektrolytisches System gebildet wird.

2. Intrauterines Pessar nach Anspruch 1, **dadurch gekennzeichnet**, daß die Drahtwicklungen auf den ersten Armen (2) weitgehend über die gesamten Längen der ersten Arme (2) reichen.

3. Intrauterines Pessar nach Anspruch 1 oder 2, **dadurch gekennzeichnet**, daß die Drahtwicklung auf dem Stiel (1) weitgehend über dessen gesamte Länge reicht.

4. Intrauterines Pessar nach einem der Ansprüche 1 bis 3, **dadurch gekennzeichnet**, daß die ersten Arme (2) über ihre gesamte Länge zum Stiel (1) hin leicht gebogen sind.

5. Intrauterines Pessar nach Anspruch 1, **dadurch gekennzeichnet**, daß die ersten Arme (2) und der Stiel (1) mit Kupfer und die zweiten Arme (3) mit Zink umwickelt sind.

6. Intrauterines Pessar nach Anspruch 1, **dadurch gekennzeichnet**, daß die ersten Arme (2) und der Stiel (1) mit Zink und die zweiten Arme (3) mit Kupfer umwickelt sind.

7. Intrauterines Pessar nach einem der Ansprüche 1 bis 6, **dadurch gekennzeichnet**, daß die Drähte einen Silber-Kern aufweisen.

8. Intrauterines Pessar nach Anspruch 5 oder 6, **dadurch gekennzeichnet**, daß die Zinkdrähte einen Silber-Kern aufweisen.

9. Intrauterines Pessar nach Anspruch 5 oder 6, **dadurch gekennzeichnet**, daß anstelle von Zink Gold vorgesehen ist.

10. Intrauterines Pessar nach einem der Ansprüche 1 bis 9, **dadurch gekennzeichnet**, daß sich in den Enden der Arme (2,3) und in dem Ende des Stiels (1), von dem die Arme (2,3) abgehen, ein Hormon befindet, das eine empfängnisverhütende Wirkung hat und über eine semipermeable Membran nach außen dringt.

## Claims

1. An intrauterine pessary for contraception, comprising a stem (1), two first arms (2) branching from one end of this stem (1) to two sides, two second arms (3) branching from the same end of the stem (1) to two sides and arranged in the angles between the first arms (2) and the stem (1), and a wire winding on the stem, **characterised in that** the second arms (3) are provided with wire windings substantially over their entire lengths and the first arms (2) have wire windings and that the metals of these wire windings and the wire winding on the stem (1) are such that an electrolytic system is formed between two neighbouring wire windings.

2. An intrauterine pessary according to claim 1, **characterised in that** the wire windings on the first arms (2) extend substantially over the entire lengths of the first arms (2).

3. An intrauterine pessary according to claim 1 or claim 2, **characterised in that** the wire winding on the stem (1) extends substantially over its entire length.

4. An intrauterine pessary according to any one of claims 1 to 3, **characterised in that** the first arms (2) are slightly bent towards the stem (1) over their entire length.

5. An intrauterine pessary according to claim 1, **characterised in that** the first arms (2) and the stem (1) are enveloped by copper and the second arms (3) are enveloped by zinc.

6. An intrauterine pessary according to claim 1, **characterised in that** the first arms (2) and the stem (1) are enveloped by zinc and the second arms (3) are enveloped by copper.

7. An intrauterine pessary according to any one of claims 1 to 6, **characterised in that** the wires have a silver core.

8. An intrauterine pessary according to claim 5 or 6, **characterised in that** the zinc wires have a silver core.

9. An intrauterine pessary according to claim 5 or 6, **characterised in that** gold is used instead of zinc.

10. An intrauterine pessary according to any one of claims 1 to 9, **characterised in that** a hormone is provided in the ends of the arms (2, 3) and in that end of the stem (1) from which the arms (2, 3) branch and that the hormone has a contraceptive action and can migrate outwardly through a semi permeable membrane.

## Revendications

1. Pessaire intra-utérin pour la contraception, constitué d'une tige (1), de deux premiers bras (2) dépassant des deux cotés de cette tige (1), à l'une de ses extrémités, de deux deuxièmes bras (3) dépassant des deux côtés de la tige (1) à la même extrémité, qui sont disposés dans les angles compris entre les premiers bras (2) et la tige (1), ainsi que d'un fil enroulé sur la tige, caractérisé en ce que les deuxièmes bras (3) présentent des fils enroulés pratiquement sur toute leur longueur et les premiers bras (2) des fils enroulés, et en ce que les métaux de ces fils enroulés et du fil enroulé sur la tige (1) sont tels qu'entre deux fils enroulés voisins il est formé un système électrolytique.

2. Pessaire intra-utérin selon la revendication 1, caractérisé en ce que les fils enroulés sur les premiers bras (2) s'étendent sur presque toute la longueur des premiers bras (2).

3. Pessaire intra-utérin selon la revendication 1 ou 2, caractérisé en ce que le fil enroulé sur la tige (1) s'étend sur presque toute sa longueur.

4. Pessaire intra-utérin selon l'une des revendications 1 à 3, caractérisé en ce que les premiers bras (2) sont légèrement arqués vers la tige (1) sur toute leur longueur.

5. Pessaire intra-utérin selon la revendication 1, caractérisé en ce que les premiers bras (2) et la tige (1) sont entourés de cuivre et les deuxièmes bras (3), de zinc.

6. Pessaire intra-utérin selon la revendication 1, caractérisé en ce que les premiers bras (2) et la tige (1) sont entourés de zinc et les deuxièmes bras (3) de cuivre.

7. Pessaire intra-utérin selon l'une des revendications 1 à 6, caractérisé en ce que les fils présentent un noyau en argent.

8. Pessaire intra-utérin selon la revendication 5 ou 6, caractérisé en ce que les fils de zinc présentent un noyau en argent.

9. Pessaire intra-utérin selon la revendication 5 ou 6, caractérisé en ce qu'au lieu du zinc il est prévu de l'or.

10. Pessaire intra-utérin selon l'une des revendications 1 à 9, caractérisé en ce que dans les extrémités des bras (2, 3) et dans l'extrémité de la tige (1) de laquelle partent les bras (2, 3), se trouve une hormone qui a un effet contraceptif et qui passe à l'extérieur par une membrane semi-perméable.
